# EUROPEAN PATENT APPLICATION

(11) **EP 4 851 134 A1**
(43) Date of publication of application: **09.12.2026**
(21) Application number: 24921002.2
(22) Date of filing: 02.02.2024
(51) Int. Cl.: C07C 213/02, C07C 215/76

(54) **PREPARATION METHOD FOR 2-AMINO-5-FLUOROPHENOL AND INTERMEDIATE THEREOF**

(71) Applicant: Max (Rudong) Chemicals Co., Ltd., Nantong, Jiangsu 226407 (CN)
(72) Inventor: CHEN, Bangchi, Nantong, Jiangsu 226407 (CN); SHENG, Qiuju, Nantong, Jiangsu 226407 (CN); SUN, Yinwei, Nantong, Jiangsu 226407 (CN); GUAN, Baochuan, Nantong, Jiangsu 226407 (CN); WANG, Zhi, Nantong, Jiangsu 226407 (CN); MA, Chaosheng, Nantong, Jiangsu 226407 (CN); ZHANG, Ning, Nantong, Jiangsu 226407 (CN)
(74) Representative: Haseltine Lake Kempner LLP
(86) International application number: PCT/CN2024/075435
(87) International publication number: WO 2025/160941

(57) **Abstract**

A method for preparing 2-amino-5-fluorophenol (I) is provided, where a compound II is reacted with a compound III to afford an intermediate IV; the intermediate IV is reacted with a compound V to yield an intermediate VI; and the intermediate VI is hydrolyzed to give the target compound 2-amino-5-fluorophenol, as shown in the following reaction scheme: 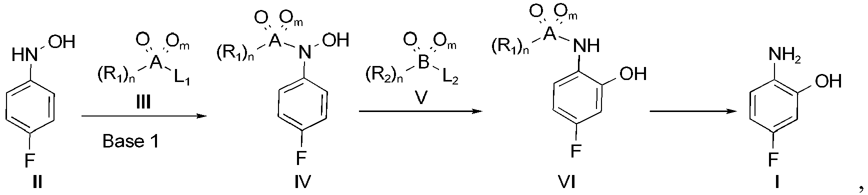 where A is C, S or P; when A is C, n is 1, and m is 0; when A is S, n is 1, and m is 0 or 1; and when A is P, n is 2, m is 0, and two R₁ groups are the same or different from each other.

## Description

### CROSS-REFERENCE TO RELATED APPLICATIONS

This application is a continuation of International Patent Application No. PCT/CN2024/075435, filed on February 2, 2024. The content of the aforementioned application, including any intervening amendments made thereto, is incorporated herein by reference in its entirety.

### TECHNICAL FIELD

This application relates to organic synthesis, and more particularly to a method for preparing 2-amino-5-fluorophenol and an intermediate therein.

### BACKGROUND

2-Amino-5-fluorophenol serves as an important chemical intermediate, and has been widely applied in the preparation of pharmaceuticals, agrochemicals and specialty materials. For example, 2-Amino-5-fluorophenol has been reported to be used in the synthesis of drugs for treating airway obstructive diseases, bronchiectasis, cystic fibrosis, asthma, emphysema and chronic obstructive pulmonary disease (WO2022042591A1, CN101014564B); drugs for treating parasitic diseases (CN105164124B); and drugs for treating and/or preventing cancer or chronic inflammation (CN103209980B). Additionally, it has also been demonstrated that 2-amino-5-fluorophenol is applicable to the preparation of highly efficient herbicides such as flumioxazin (US4792605A), thidiazimin (DE4008691A1), and trifludimoxazin (CN102459205B). Recently, 2-amino-5-fluorophenol has been used to synthesize a novel high-temperature-resistant composite material for applications in the fields of aerospace and electronic packaging (CN111393642B).

In the prior art, 2-amino-5-fluorophenol is synthesized mainly through the following five methods.

The first method involves the hydrogenation reduction of 2-nitro-5-fluorophenol in ethanol under the catalysis of a Pd/C catalyst to obtain the target compound (CN102762552B). Although this method achieves a yield greater than 99%, its starting material 5-fluoro-2-nitrophenol is not commercially available and will pose explosion risks (CN101948389A), making it unsuitable for large-scale industrial production. Furthermore, the starting material 5-fluoro-2-nitrophenol is typically produced from the hydrolysis of 2,4-difluoronitrobenzene under strong alkaline conditions (CN106083536B), however, this process suffers from poor selectivity, leading to reduced yields and increased generation of wastes.

The second method uses 6-fluorobenzoxazole as a starting material, which undergoes the oxidative ring-opening hydrolysis in the presence of platinum as catalyst and a N-bromobenzenesulfonamide salt as oxidant under alkaline conditions to afford the target product with a 93% yield (B. Laksmi et al., "An efficient platinum-catalyzed oxidation process and mechanism for the facile conversion of benzoxazoles to aminophenols", Chemical Engineering Journal, 2010, 163(3) 403-412). However, this method struggles with the use of the expensive platinum catalyst and N-bromobenzenesulfonamide salt oxidant, poor atomic efficiency and generation of large amounts of wastes, rendering it unsuitable for industrial production.

Regarding the third method, starting material 2,4-difluoroaniline undergoes reaction at 50-80°C in the presence of potassium hydroxide, subsequent acidification and steam distillation to give rise to 2-amino-5-fluorophenol (CN107459495A). Although the conversion rate of 2,4-difluoroaniline reaches 99.5% or more, the starting material 2,4-difluoroaniline is expensive and is not commercially available, and thus is not suitable for industrial production.

The fourth method uses 4-fluoronitrobenzene as starting material. This method uses zinc powder as reducing agent. In the presence of benzaldehyde and ammonium chloride, an α-phenylnitrone intermediate is produced. The α-phenylnitrone intermediate is then reacted with trichloroacetyl chloride to give the target compound 2-amino-5-fluorophenol hydrochloride (CN106496044A). However, this method suffers from tedious operations, poor overall yield (5.65%), and generation of large amounts of wastes, making it unsuitable for industrial production.

The fifth method relies on enzymatic catalysis, in which 4-fluoronitrobenzene is reduced into 4-fluorophenylhydroxylamine under the catalysis of nitroreductase, and then the 4-fluorophenylhydroxylamine is catalytically rearranged by a specially-engineered hydroxyaminobenzene mutase to generate 2-amino-5-fluorophenol (WO2014104668A1). This method struggles with poor yield (60%), and the required hydroxyaminobenzene mutase is not easily accessible, making it unsuitable for industrial production.

### SUMMARY

In order to overcome the deficiencies of the prior art, the present disclosure provides a method for preparing 2-amino-5-fluorophenol and an intermediate therein. The method provided herein features readily available raw materials, mild reaction conditions and high yield.

In a first aspect, the disclosure provides a method for preparing 2-amino-5-fluorophenol represented by formula (I), and the method comprises:
(S1) reacting a compound II with a compound III in the presence of a base 1 to generate a compound IV;
(S2) reacting the compound IV with a compound V to generate a compound VI; and
(S3) subjecting the compound VI to hydrolysis to obtain the 2-amino-5-fluorophenol, as represented by the following reaction scheme: 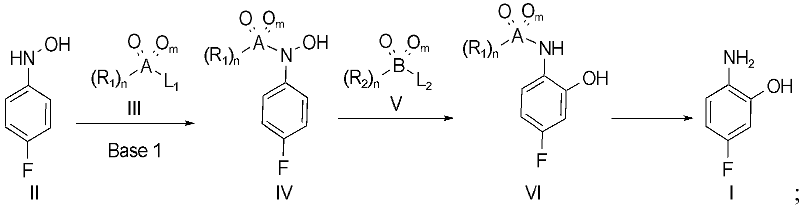
wherein A and B are each independently selected from the group consisting of a carbon atom, a sulfur atom and a phosphorus atom;
when A is the carbon atom, n is 1, and m is 0;
when A is the sulfur atom, n is 1, and m is 0 or 1;
when A is the phosphorus atom, n is 2, m is 0, and two R₁ groups are the same or different from each other;
L₁ and L₂ are each independently a leaving group; the leaving group are preferably selected from the group consisting of halogen, R₃C(=O)O- and R₃S(=O)₂O-, more preferably Cl, CH₃C(=O)O-, CCl₃C(=O)O-, ClCH₂C(=O)O-, CH₃S(=O)₂O- or CF₃S(=O)₂O-; and
R₁, R₂ and R₃ are each independently hydrogen, unsubstituted or substituted C₁-C₈ alkyl, unsubstituted or substituted C₃-C₁₀ cycloalkyl, unsubstituted or substituted C₁-C₈ alkoxy, unsubstituted or substituted C₆-C₁₂ aryl, or unsubstituted or substituted heteroaryl, preferably unsubstituted or substituted C₁-C₃ alkyl, unsubstituted or substituted C₃-C₆ cycloalkyl, unsubstituted or substituted C₁-C₃ alkoxy, unsubstituted or substituted C₆-C₁₂ aryl, or unsubstituted or substituted heteroaryl, and more preferably methyl, chloromethyl, bromomethyl, dichloromethyl, trichloromethyl, tribromomethyl, methoxy, ethoxy, chlorodifluoromethyl, bromodifluoromethyl, bromodichloromethyl, or unsubstituted or substituted phenyl.

In some embodiments, the aforementioned heteroaryl are which 1 to 4 carbon atoms in aryl are each independently substituted by an oxygen, nitrogen, or sulfur atom, such as pyrazolyl, thiazolyl, thienyl, pyridyl and pyrimidinyl.

In some embodiments, in step (S1), the base 1 is selected from the group consisting of an inorganic base, an organic base and a combination thereof; and the inorganic base is selected from the group consisting of an alkali metal carbonate, an alkali metal bicarbonate, an alkali metal phosphate, an alkali metal hydroxide, an alkali metal hydride, an alkali metal amide, an alkaline-earth metal carbonate, an alkaline-earth metal bicarbonate, an alkaline-earth metal phosphate, an alkaline-earth metal hydroxide, an alkaline-earth metal oxide, an alkaline-earth metal hydride and an alkaline-earth metal amide, preferably an alkali metal carbonate, an alkali metal bicarbonate, an alkali metal hydroxide, an alkaline-earth metal carbonate, an alkaline-earth metal bicarbonate or an alkaline-earth metal hydroxide, and more preferably sodium carbonate, sodium bicarbonate, potassium carbonate, potassium bicarbonate, sodium hydroxide or potassium hydroxide.

In some embodiments, in step (S1), the organic base is selected from the group consisting of an organic salt, an organic amine and a combination thereof; the organic salt is selected from the group consisting of an alkyl carboxylate, an aryl carboxylate, an alkoxide, a metal alkyl and a metal aryl; the organic amine is selected from the group consisting of an alkyl amine and an aryl amine; the alkyl amine is preferably as a tertiary amine, more preferably triethylamine; and the aryl amine is preferably as a pyridinamine compound, more preferably pyridine or methylpyridine

In some embodiments, the compound III in step (S1) and the compound V in step (S2) are each independently an acyl halide, an acid anhydride or a chloroformate; the acyl halide is selected from the group consisting of unsubstituted and substituted C₁-C₈ alkyl acyl halide, unsubstituted and substituted C₁-C₈ alkylsulfonyl halide, unsubstituted and substituted C₃-C₁₀ cycloalkyl acyl halide, unsubstituted and substituted C₆-C₁₂ aryl acyl halide, unsubstituted and substituted C₆-C₁₂ sulfonyl halide, alkylphosphoryl halide, arylphosphoryl halide, and unsubstituted and substituted heteroaryl acyl halide, preferably acetyl chloride, chloroacetyl chloride, bromoacetyl chloride, dichloroacetyl chloride, trichloroacetyl chloride, tribromoacetyl chloride, acetic anhydride, chloroacetic anhydride, dichloroacetic anhydride, trichloroacetic anhydride, bromoacetic anhydride, methyl chloroformate, ethyl chloroformate, 2-chloro-2,2-difluoroacetyl chloride, 2-bromo-2,2-difluoroacetyl chloride, 2-bromo-2,2-dichloroacetyl chloride, benzenesulfonyl chloride, benzenesulfonic anhydride, p-toluenesulfonyl chloride, p-toluenesulfonic anhydride, methanesulfonyl chloride, methanesulfonic anhydride, dimethylphosphoryl chloride, or diarylphosphoryl chloride.

In some embodiments, step (S1) is performed at -20-40°C, preferably -15-25°C, and more preferably -15-5°C.

In some embodiments, in step (S1), a molar ratio of the compound II to the base 1 is 1:1-2, preferably 1:1-1.5, and more preferably 1:1-1.2.

In some embodiments, in step (S1), a molar ratio of the compound II to the compound III is 1:1-2, preferably 1:1-1.5, and more preferably 1:1-1.2.

In some embodiments, step (S1) is performed in a solvent 1; the solvent 1 is an organic solvent or a mixture thereof with water; the organic solvent is selected from the group consisting of an aromatic hydrocarbon solvent, an amide solvent, a (sulfoxide)sulfone solvent, a halogenated hydrocarbon solvent, an ether solvent, an ester solvent and a combination thereof; the aromatic hydrocarbon solvent is a C₆-C₁₂ aromatic hydrocarbon solvent, preferably toluene, xylene or trimethylbenzene; the amide solvent is a C₁-C₆ amide solvent, preferably N,N-dimethylformamide or N,N-dimethylacetamide; the (sulfoxide)sulfone solvent is a C₁-C₆ (sulfoxide)sulfone solvent, preferably dimethyl sulfoxide or sulfolane; the halogenated hydrocarbon solvent is dichloromethane, chloroform, carbon tetrachloride or dichloroethane; the ether solvent is methyl tert-butyl ether, tetrahydrofuran or 2-methyltetrahydrofuran; and the ester solvent is ethyl acetate.

In an embodiment, the solvent 1 is toluene, tetrahydrofuran, methyltetrahydrofuran or ethyl acetate.

In some embodiments, step (S2) is performed at 20-120°C, preferably 30-80°C, and more preferably 40-60°C.

In some embodiments, in step (S2), a molar ratio of the compound IV to the compound V is 1:1-2, preferably 1:1-1.5, and more preferably 1:1-1.2.

In some embodiments, step (S2) is performed in a solvent 2; the solvent 2 is an organic solvent or a mixture thereof with water; the organic solvent is selected from the group consisting of an aromatic hydrocarbon solvent, an amide solvent, a (sulfoxide)sulfone solvent, a halogenated hydrocarbon solvent, an ether solvent, an ester solvent and a combination thereof; the aromatic hydrocarbon solvent is C₆-C₁₂ aromatic hydrocarbon solvent, preferably toluene, xylene or trimethylbenzene; the amide solvent is C₁-C₆ amide solvent, preferably N,N-dimethylformamide or N,N-dimethylacetamide; the (sulfoxide)sulfone solvent is C₁-C₆ (sulfoxide)sulfone solvent, preferably dimethyl sulfoxide or sulfolane; the halogenated hydrocarbon solvent is dichloromethane, chloroform, carbon tetrachloride or dichloroethane; the ether solvent is methyl tert-butyl ether, tetrahydrofuran or 2-methyltetrahydrofuran; and the ester solvent is ethyl acetate.

In an embodiment, the solvent 2 is a halogenated alkane solvent, the aromatic hydrocarbon solvent, acetonitrile or tetrahydrofuran.

In some embodiments, in step (S3), the hydrolysis is performed in the presence of an acid or a base 2.

In some embodiments, the acid is a protic acid or a Lewis acid; the protic acid is selected from the group consisting of hydrofluoric acid, hydrochloric acid, sulfuric acid and phosphoric acid; and the Lewis acid is selected from the consisting of AlCl₃, FeCl₃, SbCl₅, SnCl₄, BCl₃, TiCl₄, ZnCl₂, MgCl₂, alkyl aluminum and alkyl boron.

In an embodiment, the acid is the protic acid, preferably hydrochloric acid or sulfuric acid.

In some embodiments, the base 2 is an inorganic base, an organic base or a combination thereof; the inorganic base is selected from the group consisting of an alkali metal hydroxide, an alkali metal carbonate, an alkali metal bicarbonate, an alkaline-earth metal hydroxide, an alkaline-earth metal oxide, an alkaline-earth metal carbonate and an alkaline-earth metal bicarbonate, preferably an alkali metal hydroxide or an alkaline-earth metal hydroxide, and more preferably sodium hydroxide or potassium hydroxide; and the organic base is an organic amine, preferably a primary amine or a secondary amine.

In some embodiments, step (S3) is performed at 20-120°C, preferably 50-120°C, and more preferably 80-100°C.

In some embodiments, in step (S3), a molar ratio of the compound VI to the acid is 1:1-2, preferably 1:1-1.5, and more preferably 1:1-1.2.

In some embodiments, step (S3) is optionally performed in a solvent 3; the solvent 3 is preferably as a polar solvent selected from the group consisting of an alcohol solvent, a (sulfoxide)sulfone solvent, an amide solvent, water and a combination thereof; the alcohol solvent is a C₁-C₁₀ alcohol solvent, preferably methanol, ethanol, n-propanol, isopropanol, butanol, octanol, ethylene glycol, propylene glycol or glycerol; the (sulfoxide)sulfone solvent is a C₁-C₆ (sulfoxide)sulfone solvent, preferably dimethyl sulfoxide or sulfolane; and the amide solvent is a C₁-C₆ amide solvent, preferably N-methylpyrrolidone, N,N-dimethylformamide or N,N-dimethylacetamide.

In some embodiments, the 2-Amino-5-fluorophenol is prepared from the compound II in a one-pot manner.

In a second aspect, the present disclosure provides a compound represented by formula (IV): 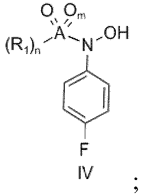
wherein A is selected from the group consisting of a carbon atom, a sulfur atom and a phosphorus atom;
when A is the carbon atom, n is 1, m is 0, and R₁ is unsubstituted C₂-C₈ alkyl, substituted C₁-C₈ alkyl, unsubstituted or substituted C₃-C₁₀ cycloalkyl, unsubstituted or substituted C₁-C₈ alkoxy, unsubstituted or substituted C₆-C₁₂ aryl, or unsubstituted or substituted heteroaryl, preferably halogen-substituted C₁-C₈ alkyl, or unsubstituted or substituted C₆-C₁₂ aryl, and more preferably chloromethyl, bromomethyl, dichloromethyl, trichloromethyl, tribromomethyl, chlorodifluoromethyl, bromodifluoromethyl, or substituted phenyl;
when A is the sulfur atom, n is 1, and m is 0 or 1;
when A is the phosphorus atom, n is 2, m is 0, and two R₁ groups are each independently hydrogen, unsubstituted or substituted C₁-C₈ alkyl, unsubstituted or substituted C₁-C₈ alkoxy, unsubstituted or substituted C₆-C₁₂ aryl, or unsubstituted or substituted heteroaryl, preferably unsubstituted or substituted C₁-C₃ alkyl, unsubstituted or substituted C₁-C₃ alkoxy, or unsubstituted or substituted C₆-C₁₂ aryl, and more preferably methyl, chloromethyl, bromomethyl, dichloromethyl, trichloromethyl, tribromomethyl, methoxy, ethoxy, chlorodifluoromethyl, bromodifluoromethyl, bromodichloromethyl, or unsubstituted or substituted phenyl.
n R₁ groups are the same or different from each other;
when A is the sulfur atom, n is 1 and m is 0, R₁ is hydrogen, unsubstituted or substituted C₁-C₈ alkyl, unsubstituted or substituted C₃-C₁₀ cycloalkyl, unsubstituted or substituted C₁-C₈ alkoxy, unsubstituted or substituted C₆-C₁₂ aryl, or unsubstituted or substituted heteroaryl, preferably unsubstituted or substituted C₁-C₃ alkyl, unsubstituted or substituted C₁-C₃ alkoxy, or unsubstituted or substituted C₆-C₁₂ aryl, and more preferably methyl, chloromethyl, bromomethyl, dichloromethyl, trichloromethyl, tribromomethyl, methoxy, ethoxy, chlorodifluoromethyl, bromodifluoromethyl, bromodichloromethyl, or unsubstituted or substituted phenyl; and
when A is the sulfur atom, n is 1 and m is 1, R₁ is unsubstituted C₂-C₈ alkyl, substituted C₁-C₈ alkyl, unsubstituted or substituted C₁-C₈ alkoxy, unsubstituted or substituted C₆-C₁₂ aryl, or unsubstituted or substituted heteroaryl, preferably unsubstituted C₂-C₃ alkyl, substituted C₁-C₃ alkyl, unsubstituted or substituted C₁-C₃ alkoxy, or unsubstituted or substituted C₆-C₁₂ aryl, and more preferably chloromethyl, bromomethyl, dichloromethyl, trichloromethyl, tribromomethyl, methoxy, ethoxy, chlorodifluoromethyl, bromodifluoromethyl, bromodichloromethyl, or unsubstituted or substituted phenyl.

Compared to the prior art, the present disclosure has the following beneficial effects.
(1) The present disclosure provides a novel strategy for preparing 2-amino-5-fluorophenol and a novel intermediate.
(2) The raw and auxiliary materials involved in the preparation are readily available, and no special reaction equipment is required.
(3) The preparation method features mild reaction conditions and convenient operation.
(4) The preparation process is highly safe, with low waste generation and high yield.
(5) The preparation method is suitable for the large-scale industrial production.

### DETAILED DESCRIPTION OF EMBODIMENTS

The term "halogen" as used herein refers to fluorine, chlorine, bromine, and iodine.

As used herein, the term "substituted" refers to substitution in which one or more hydrogen atoms on a carbon atom or a nitrogen atom are independently substituted by halogen, C₁-C₆ alkyl, C₁-C₆ haloalkyl, C₃-C₉ cycloalkyl, C₃-C₉ halocycloalkyl, C₂-C₆ alkenyl, C₂-C₆ haloalkenyl, C₂-C₆ alkynyl, C₂-C₆ haloalkynyl, C₁-C₆ alkoxy, C₁-C₆ haloalkoxy, C₁-C₆ alkylthio, C₁-C₆ haloalkylthio, C₁-C₆ alkylsulfinyl, C₁-C₆ haloalkylsulfinyl, C₁-C₆ alkylsulfonyl, C₁-C₆ haloalkylsulfonyl, C₂-C₇ alkylcarbonyl, C₂-C₇ haloalkylcarbonyl, C₂-C₇ alkylcarbonyloxy, C₂-C₇ haloalkylcarbonyloxy, C₁-C₆ alkylsulfonyloxy, C₁-C₆ haloalkylsulfonyloxy, C₂-C₇ alkoxycarbonyl, C₂-C₇ haloalkoxycarbonyl, C₂-C₇ alkylcarbonylamino, C₂-C₇ haloalkylcarbonylamino, C₂-C₇ alkoxycarbonylamino, C₂-C₇ haloalkoxycarbonylamino, C₂-C₇ alkylaminocarbonyl, C₂-C₇ haloalkylaminocarbonyl, C₁-C₆ alkylamino, C₁-C₆ haloalkylamino, C₂-C₆ alkenylamino, C₂-C₆ haloalkenylamino, C₂-C₆ alkynylamino, C₂-C₆ haloalkynylamino, C₃-C₉ cycloalkylamino, C₃-C₉ halocycloalkylamino, C₃-C₇ alkenylaminocarbonyl, C₃-C₇ haloalkenylaminocarbonyl, C₃-C₇ alkynylaminocarbonyl, C₃-C₇ haloalkynylaminocarbonyl, C₄-C₁₀ cycloalkylaminocarbonyl, C₄-C₁₀ halocycloalkylaminocarbonyl, amino, carbamoyl, sulfamoyl, cyano, nitro, hydroxyl, carboxyl, optionally substituted phenyl, optionally substituted heterocyclyl, optionally substituted benzyl, optionally substituted phenylcarbonyl, or optionally substituted phenylamino.

The technical solutions of the present disclosure will be described clearly and completely below with reference to the following embodiments. Obviously, described herein are merely some embodiments of the present disclosure, rather than all embodiments of the present disclosure. Based on the embodiments in the present disclosure, all other embodiments obtained by those of ordinary skill in the art without making creative effort shall fall within the scope of the present disclosure defined by the appended claims.

### Example 1

18.45 g of 4-fluorophenylhydroxylamine was dissolved in 40 mL of tetrahydrofuran. The mixture was cooled to a temperature ranging from -20°C to -15°C. 19 g of triethylamine was added followed by 17 g of chloroacetyl chloride. The mixture was reacted at about -15°C until the reaction was completed as monitored by in-process control. After usual workup,29.5 g of 2-chloro-N-(4-fluorophenyl)-N-hydroxyacetamide (97% yield) was obtained. ¹H NMR (CDCl₃, 500 MHz, TMS) δ 10.73 (s, 1H), 6.90-6.87 (m, 2H), 6.82-6.78 (m, 2H), 4.59 (s, 2H).

### Example 2

17 g of 4-fluorophenylhydroxylamine was dissolved in 40 mL of ethyl acetate. The mixture was cooled to a temperature ranging from -5°C to 0°C. 16.8 g of chloroacetyl chloride was added dropwise followed by 145.6 g of a 10% aqueous sodium bicarbonate solution. The mixture was reacted at the same temperature ranging from -5°C to 0°C until the reaction was completed. After usual workup, 26.2 g of 2-chloro-N-(4-fluorophenyl)-N-hydroxyacetamide (98% yield) was obtained.

### Example 3

12 g of 4-fluorophenylhydroxylamine was dissolved in 30 mL of methyltetrahydrofuran. The mixture was cooled to a temperature ranging from -20°C to -15°C. 10.5 g of triethylamine was added followed by 7.9 g of acetyl chloride. The mixture was reacted at about -15°C until the reaction was completed as monitored by in-process control. After usual workup, 16.2 g of N-(4-fluorophenyl)-N-hydroxyacetamide (96% yield) was obtained.

### Example 4

17 g of 4-fluorophenylhydroxylamine was dissolved in 40 mL of toluene. The mixture was cooled to a temperature ranging from -20°C to -15°C.13.6 g of acetyl chloride was added dropwise followed by 17.04 g of triethylamine. The mixture was reacted at the temperature ranging from -20°C to -15°C until the reaction was completed. After usual workup,22 g of N-(4-fluorophenyl)-N-hydroxyacetamide (97% yield) was obtained. ¹H NMR (CDCl₃, 500 MHz, TMS) δ 10.47 (s, 1H), 7.68-7.60 (m, 2H), 7.25-7.16 (m, 2H), 2.18 (s, 3H).

### Example 5

17 g of 4-fluorophenylhydroxylamine was dissolved in 30 mL of dichloromethane. The mixture was cooled to a temperature ranging from -20°C to 15°C. 18.8 g of ethyl chloroformate was added dropwise followed by 13.3 g of pyridine. The reaction was maintained at a temperature range of -20°C to 15°C until the reaction was completed. After usual workup 26 g of ethyl N-(4-fluorophenyl)-N-hydroxycarbamate (93% yield) was obtained. ¹H NMR (CDCl₃, 500 MHz, TMS) δ 7.43-7.22 (m, 2H), 7.19-7.08 (m, 2H), 4.33 (q, *J* = 7.0 Hz, 2H), 1.33 (t, *J* = 7.0 Hz, 3H).

### Example 6

17 g of 4-fluorophenylhydroxylamine was dissolved in 30 mL of dimethyl sulfoxide. The mixture was cooled to a temperature ranging from -20°C to 15°C. 30.6 g of benzenesulfonyl was added dropwise followed by13.3 g of pyridine. The reaction was maintained at a temperature range of -20°C to 15°C until the reaction was completed. After usual workup,34 g of N-(4-fluorophenyl)-N-hydroxybenzenesulfonamide (95% yield) was obtained. ¹H NMR (CDCl₃, 500 MHz, TMS) δ 7.66-7.62 (m, 1H), 7.56-7.54 (m, 2H), 7.46-7.43 (m, 2H), 7.13-7.10 (m, 2H), 6.96-6.92 (m, 2H).

### Example 7

17 g of 4-fluorophenylhydroxylamine was dissolved in 30 mL of sulfolane. The mixture was cooled to a temperature ranging from -20°C to -15°C.19.8 g of methanesulfonyl chloride was added dropwise followed by 13.3 g of pyridine. The reaction was maintained at a temperature range of -20°C to -15°C until the reaction was completed. After usual workup, 25 g of N-(4-fluorophenyl)-N-hydroxymethanesulfonamide (96% yield) was obtained. ¹H NMR (CDCl₃, 500 MHz, TMS) δ 7.50-7.47 (m, 2H), 7.10-7.06 (m, 2H), 2.86 (s, 3H).

### Example 8

20.3 g of 2-chloro-N-(4-fluorophenyl)-N-hydroxyacetamide prepared in Example 1 or 2 was dissolved in 50 mL of chloroform, purged with nitrogen, heated to 45°C-50°C, added with 21.35 g of trichloroacetyl chloride. The mixture was reacted until the reaction was completed as monitored by in-process control. After usual workup 19.3 g of 2-chloro-N-(4-fluoro-2-hydroxyphenyl) acetamide (95% yield) was obtained.

### Example 9

8.5 g of N-(4-fluorophenyl)-N-hydroxyacetamide prepared in Example 3 or 4 was dissolved in 50 mL of toluene, purged with nitrogen, heated to 50°C, added with 17 g of trichloroacetic anhydride. The mixture was reacted until the reaction was completed as monitored by in-process control. After usual workup, 8 g of N-(4-fluoro-2-hydroxyphenyl)acetamide (95% yield) was obtained.

### Example 10

The target compound prepared in Example 6 was subjected to the same procedure as in Example 8 or 9. N-(4-fluoro-2-hydroxyphenyl) benzenesulfonamide (92% yield) was obtained. ¹H NMR (500 MHz, DMSO) δ 10.02 (s, 1H), 9.32 (s, 1H), 7.70 (d, *J* = 7.5 Hz, 2H), 7.60 (t, *J* = 7.3 Hz, 1H), 7.52 (t, *J* = 7.6 Hz, 2H), 7.09 (dd, *J* = 8.6, 6.5 Hz, 1H), 6.60-6.47 (m, 2H).

### Example 11

The target compound prepared in Example 7 was subjected to the same procedure as in Example 8 or 9. N-(4-fluoro-2-hydroxyphenyl) methanesulfonamide (90% yield) was obtained. ¹H NMR (500 MHz, DMSO) δ 10.36 (s, 1H), 8.77 (s, 1H), 7.17 (dd, *J* = 8.6, 6.5 Hz, 1H), 6.72-6.56 (m, 2H), 2.91 (s, 3H).

### Example 12

19.3 g of the target compound prepared in Example 8 was added to 40 g of a 10% hydrochloric acid solution, heated to 80°C, stirred and reacted until the reaction was completed as monitored by in-process control. After usual workup, 11.8 g of 2-amino-5-fluorophenol (98% yield) was obtained.

### Example 13

8 g of the target compound prepared in Example 9 was added to 20 g of a 10% sulfuric acid solution, heated to 100°C, stirred and reacted until the reaction was completed as monitored by in-process control. After usual workup,6.1 g of 2-amino-5-fluorophenol (96% yield) was obtained.

### Example 14

20.3 g of 2-chloro-N-(4-fluorophenyl)-N-hydroxyacetamide prepared according to Example 1 was dissolved in 50 mL of ethyl acetate, heated to 45-55°C under a nitrogen atmosphere, added with trichloroacetyl chloride to continue the reaction. After the reaction was completed as monitored by in-process control, the reaction mixture was added with 40 g of a 10% hydrochloric acid solution, heated under reflux until the reaction was completed. After usual workup, 10.5 g of 2-amino-5-fluorophenol (82% yield) was obtained.

It should be noted that the above embodiments are only used to illustrate the technical solutions of the present disclosure, rather than limiting the disclosure. Although the present disclosure has been described in detail with reference to the above embodiments, those of ordinary skill in the art could still make various modifications and substitutions to the technical solutions recited in the above embodiments. It should be understood that modifications or substitutions made without departing from the spirit of the disclosure shall fall within the scope of the present disclosure defined by the appended claims.

## Claims

1. A method for preparing 2-amino-5-fluorophenol represented by formula (I), comprising:
(S1) reacting a compound II with a compound III in the presence of a base 1 to generate a compound IV;
(S2) reacting the compound IV with a compound V to generate a compound VI; and
(S3) subjecting the compound VI to hydrolysis to obtain the 2-amino-5-fluorophenol, as represented by the following reaction scheme: 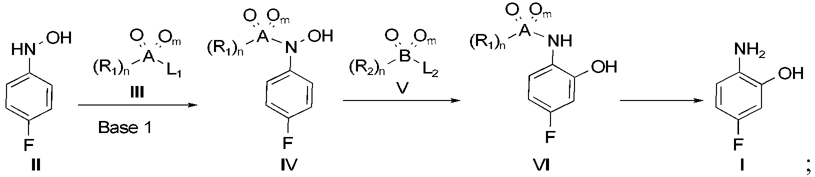
**characterized in that** A and B are each independently selected from the group consisting of a carbon atom, a sulfur atom and a phosphorus atom;
when A is the carbon atom, n is 1, and m is 0;
when A is the sulfur atom, n is 1, and m is 0 or 1;
when A is the phosphorus atom, n is 2, m is 0, and two R₁ groups are the same or different from each other;
L₁ and L₂ are each independently a leaving group; and the leaving group is selected from the group consisting of halogen, R₃C(=O)O- and R₃S(=O)₂O-; and
R₁, R₂ and R₃ are each independently hydrogen, unsubstituted or substituted C₁-C₈ alkyl, unsubstituted or substituted C₃-C₁₀ cycloalkyl, unsubstituted or substituted C₁-C₈ alkoxy, unsubstituted or substituted C₆-C₁₂ aryl, or unsubstituted or substituted heteroaryl.

2. The method according to claim 1, **characterized in that** R₁, R₂ and R₃ are each independently unsubstituted or substituted C₁-C₃ alkyl, unsubstituted or substituted C₃-C₆ cycloalkyl, unsubstituted or substituted C₁-C₃ alkoxy, unsubstituted or substituted C₆-C₁₂ aryl, or unsubstituted or substituted heteroaryl.

3. The method according to claim 2, **characterized in that** R₁, R₂ and R₃ are each independently methyl, chloromethyl, bromomethyl, dichloromethyl, trichloromethyl, tribromomethyl, methoxy, ethoxy, chlorodifluoromethyl, bromodifluoromethyl, bromodichloromethyl, unsubstituted or substituted phenyl, unsubstituted or substituted pyrazolyl, unsubstituted or substituted thiazolyl, unsubstituted or substituted thienyl, unsubstituted or substituted pyridyl, or unsubstituted or substituted pyrimidinyl.

4. The method according to claim 1, **characterized in that** in step (S1), the first base is selected from the group consisting of an inorganic base, an organic base and a combination thereof; and
in step (S3), the hydrolysis is performed in the presence of an acid or a base 2.

5. The method according to claim 4, **characterized in that** in step (S1), the inorganic base is selected from the group consisting of an alkali metal carbonate, an alkali metal bicarbonate, an alkali metal phosphate, an alkali metal hydroxide, an alkali metal hydride, an alkali metal amide, an alkaline-earth metal carbonate, an alkaline-earth metal bicarbonate, an alkaline-earth metal phosphate, an alkaline-earth metal hydroxide, an alkaline-earth metal hydride and an alkaline-earth metal amide; the organic base is selected from the group consisting of an organic salt, an organic amine and a combination thereof; and
in step (S3), the acid is a protic acid or a Lewis acid; and the base 2 is an inorganic base, an organic base or a combination thereof.

6. The method according to claim 5, **characterized in that** in step (S1), the inorganic base is selected from the group consisting of an alkali metal carbonate, an alkali metal bicarbonate, an alkali metal hydroxide, an alkaline-earth metal carbonate, an alkaline-earth metal bicarbonate and an alkaline-earth metal hydroxide; the organic salt is selected from the group consisting of an alkyl carboxylate, an aryl carboxylate, an alkoxide, a metal alkyl and a metal aryl; the organic amine is selected from the group consisting of an alkyl amine and an aryl amine; and
in step (S3), the protic acid is selected from the group consisting of hydrofluoric acid, hydrochloric acid, sulfuric acid and phosphoric acid; the Lewis acid is selected from the consisting of AlCl₃, FeCl₃, SbCl₅, SnCl₄, BCl₃, TiCl₄, ZnCl₂, MgCl₂, alkyl aluminum and alkyl boron; the inorganic base is selected from the group consisting of an alkali metal hydroxide, an alkali metal carbonate, an alkali metal bicarbonate, an alkaline-earth metal hydroxide, an alkaline-earth metal carbonate and an alkaline-earth metal bicarbonate; and the organic base is an organic amine.

7. The method according to claim 6, **characterized in that** in step (S1), the inorganic base is selected from the group consisting of sodium carbonate, sodium bicarbonate, potassium carbonate, potassium bicarbonate, sodium hydroxide and potassium hydroxide; the alkyl amine is a tertiary amine, preferably triethylamine; and the aryl amine is a pyridinamine compound, preferably pyridine or methylpyridine; and
in step (S3), the acid is a protic acid, preferably hydrochloric acid or sulfuric acid; and the organic base is a primary amine or a secondary amine.

8. The method according to claim 1, **characterized in that** the 2-amino-5-fluorophenol is prepared from the compound II in a one-pot manner.

9. A compound, represented by formula (IV): 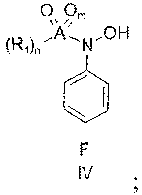
**characterized in that** A is selected from the group consisting of a carbon atom, a sulfur atom and a phosphorus atom;
when A is the carbon atom, n is 1, m is 0, and R₁ is unsubstituted C₂-C₈ alkyl, substituted C₁-C₈ alkyl, unsubstituted or substituted C₃-C₁₀ cycloalkyl, unsubstituted or substituted C₁-C₈ alkoxy, unsubstituted or substituted C₆-C₁₂ aryl, or unsubstituted or substituted heteroaryl;
when A is the sulfur atom, n is 1, m is 0 or 1;
when A is the phosphorus atom, n is 2, m is 0, and two R₁ groups are the same or different from each other; wherein the two R₁ groups are each independently hydrogen, unsubstituted or substituted C₁-C₈ alkyl, unsubstituted or substituted C₁-C₈ alkoxy, unsubstituted or substituted C₆-C₁₂ aryl, or unsubstituted or substituted heteroaryl;
when A is the sulfur atom, n is 1 and m is 0, R₁ is hydrogen, unsubstituted or substituted C₁-C₈ alkyl, unsubstituted or substituted C₃-C₁₀ cycloalkyl, unsubstituted or substituted C₁-C₈ alkoxy, unsubstituted or substituted C₆-C₁₂ aryl, or unsubstituted or substituted heteroaryl; and
when A is the sulfur atom, n is 1 and m is 1, R₁ is unsubstituted C₂-C₈ alkyl, substituted C₁-C₈ alkyl, unsubstituted or substituted C₁-C₈ alkoxy, unsubstituted or substituted C₆-C₁₂ aryl, or unsubstituted or substituted heteroaryl.

10. The compound according to claim 9, **characterized in that** when A is the phosphorus atom, the two R₁ groups are each independently unsubstituted or substituted C₁-C₃ alkyl, unsubstituted or substituted C₁-C₃ alkoxy, or unsubstituted or substituted C₆-C₁₂ aryl;
when A is the carbon atom, R₁ is halogen-substituted C₁-C₈ alkyl, or unsubstituted or substituted C₆-C₁₂ aryl; preferably, R₁ is selected from the group consisting of chloromethyl, bromomethyl, dichloromethyl, trichloromethyl, tribromomethyl, methoxy, ethoxy, chlorodifluoromethyl, bromodifluoromethyl, bromodichloromethyl and substituted phenyl;
when A is the sulfur atom, n is 1 and m is 0, R₁ is unsubstituted or substituted C₁-C₃ alkyl, unsubstituted or substituted C₁-C₃ alkoxy, or unsubstituted or substituted C₆-C₁₂ aryl; preferably, R₁ is selected from the group consisting of methyl, chloromethyl, bromomethyl, dichloromethyl, trichloromethyl, tribromomethyl, methoxy, ethoxy, chlorodifluoromethyl, bromodifluoromethyl, bromodichloromethyl, unsubstituted phenyl and substituted phenyl; and
when A is the sulfur atom, n is 1 and m is 1, R₁ is unsubstituted C₂-C₃ alkyl, substituted C₁-C₃ alkyl, unsubstituted or substituted C₁-C₃ alkoxy, or unsubstituted or substituted C₆-C₁₂ aryl; preferably, R₁ is selected from the group consisting of chloromethyl, bromomethyl, dichloromethyl, trichloromethyl, tribromomethyl, methoxy, ethoxy, chlorodifluoromethyl, bromodifluoromethyl, bromodichloromethyl, unsubstituted phenyl and substituted phenyl.
